(19) Europäisches Patentamt
European Patent Office
Office européen des brevets

(11) **EP 3 999 895 B1**

(12) **FASCICULE DE BREVET EUROPEEN**

(45) Date de publication et mention de la délivrance du brevet:
**05.03.2025 Bulletin 2025/10**

(21) Numéro de dépôt: **20753993.3**

(22) Date de dépôt: **17.07.2020**

(51) Classification Internationale des Brevets (IPC):
**G02B 13/00** $^{(2006.01)}$

(52) Classification Coopérative des Brevets (CPC):
**G02B 17/02; G01J 3/0208; G01J 3/024; G01N 33/386; G02B 27/0012**

(86) Numéro de dépôt international:
**PCT/FR2020/051294**

(87) Numéro de publication internationale:
**WO 2021/009469 (21.01.2021 Gazette 2021/03)**

(54) **PROCÉDÉ DE FABRICATION D'UN DISPOSITIF DE MESURE OPTIQUE APTE À FONCTIONNER SOUS RAYONNEMENT IONISANT ET DISPOSITIF DE MESURE OPTIQUE APTE À FONCTIONNER SOUS RAYONNEMENT IONISANT**

VERFAHREN ZUR HERSTELLUNG EINER UNTER IONISIERENDER STRAHLUNG ARBEITENDEN OPTISCHEN MESSVORRICHTUNG UND UNTER IONISIERENDER STRAHLUNG ARBEITENDE OPTISCHE MESSVORRICHTUNG

METHOD FOR MANUFACTURING AN OPTICAL MEASURING DEVICE CAPABLE OF OPERATING UNDER IONISING RADIATION AND OPTICAL MEASURING DEVICE CAPABLE OF OPERATING UNDER IONISING RADIATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.07.2019 FR 1908146**

(43) Date de publication de la demande:
**25.05.2022 Bulletin 2022/21**

(73) Titulaires:
• **Agence Nationale pour la Gestion des Déchets Radioactifs**
  **92298 Châtenay-Malabry (FR)**
• **Université Jean Monnet Saint-Étienne**
  **42100 Saint-Étienne (FR)**
• **Centre National de la Recherche Scientifique**
  **75794 Paris Cedex 16 (FR)**
• **OPTSYS**
  **78034 Versailles Cedex (FR)**

(72) Inventeurs:
• **GIRARD, Sylvain**
  **42530 SAINT-GENEST LERPT (FR)**
• **ALLANCHE, Timothé**
  **42000 SAINT-ETIENNE (FR)**
• **BOUKENTER, Aziz**
  **42400 SAINT-ETIENNE (FR)**

(74) Mandataire: **Brevalex**
  **Tour Trinity**
  **1 B Place de la Défense**
  **92400 Courbevoie (FR)**

(56) Documents cités:
EP-A1- 1 876 150    CN-A- 105 676 451
CN-A- 105 676 451    US-A- 4 620 863

• MCGRATH B ET AL: "EFFECTS OF NUCLEAR RADIATION ON THE OPTICAL PROPERTIES OF CERIUM-DOPED GLASS", NUCLEAR INSTRUMENTS AND METHODS,, vol. 135, no. 1, 1 May 1976 (1976-05-01), pages 93 - 97, XP001429817

- **ABOUHASWA A S ET AL: "Synthesis, structure, optical and gamma radiation shielding properties of B2O3-PbO2-Bi2O3 glasses", COMPOSITES PART B, ELSEVIER, AMSTERDAM, NL, vol. 172, 7 May 2019 (2019-05-07), pages 218 - 225, XP085744626, ISSN: 1359-8368, [retrieved on 20190507], DOI: 10.1016/J.COMPOSITESB.2019.05.040**
- **MCGRATH B ET AL: "EFFECTS OF NUCLEAR RADIATION ON THE OPTICAL PROPERTIES OF CERIUM-DOPED GLASS", NUCLEAR INSTRUMENTS AND METHODS,, vol. 135, no. 1, 1 May 1976 (1976-05-01), pages 93 - 97, XP001429817**
- **ABOUHASWA A S ET AL: "Synthesis, structure, optical and gamma radiation shielding properties of B2O3-PbO2-Bi2O3 glasses", COMPOSITES:PART B, ELSEVIER, AMSTERDAM, NL, vol. 172, 7 May 2019 (2019-05-07), pages 218 - 225, XP085744626, ISSN: 1359-8368, [retrieved on 20190507], DOI: 10.1016/J.COMPOSITESB.2019.05.040**

## Description

### Domaine technique

L'invention concerne le domaine de la mesure optique telle que l'imagerie dans le visible et concerne plus précisément le domaine de la mesure optique en milieu ionisant.

L'invention concerne ainsi un procédé de fabrication d'un dispositif de mesure optique et un dispositif de mesure optique.

### État de l'art antérieur

Les mesures optiques en milieu ionisant, tel qu'un lieu de stockage de déchets radioactifs, nécessitent la conception de dispositifs de mesure optique aptes à fonctionner sous les rayonnements ionisants qui peuvent exister dans un tel milieu. On notera que cette problématique est particulièrement présente pour les doses d'irradiation supérieures à 100 kGy, voire 200 kGy ou encore 500 kGy.

En effet, dans le cas d'un dispositif de mesure optique non adapté, et notamment en ce qui concerne les dispositifs de mesure optique polychromatiques se trouvant dans un tel milieu, on observe notamment deux phénomènes :

- une dégradation du système de détection, tel qu'un capteur semiconducteur, sous l'effet des rayonnements ionisants,
- une dégradation des caractéristiques de transmission des composants optiques de son système optique sous l'effet des rayonnements ionisants, cette dégradation pouvant aller d'une opacification partielle à totale desdits composants optiques.

On entend ci-dessus et dans le reste de ce document par dispositif de mesure optique polychromatique et système optique polychromatique, les dispositifs de mesure optique et les systèmes optiques qui mettent en œuvre des rayonnements polychromatiques dans le cadre de leur utilisation normale. Ainsi, si de tels dispositifs de mesure optique peuvent être des dispositifs de mesure optique offrant une mesure polychromatique en tant que telle, telle qu'une image RGB, ils peuvent également être des dispositifs fournissant une mesure « monochromatique » c'est-à-dire non distinctive en longueur d'onde, comme une image noir et blanc.

Il est entendu, ci-dessus et dans le reste du présent document, par « composant optique » un composant optique, tel qu'une lentille, un prisme ou un filtre, utilisé pour modifier la trajectoire d'un rayonnement optique ou la composition du rayonnement. Cette modification de trajectoire ou de composition peut être soit une modification en tant que telle, telle qu'une modification du chemin optique dudit rayonnement optique, une modification de la forme du rayonnement, telle qu'une convergence ou une divergence dudit rayonnement, une modification de la répartition des composantes du rayonnement, tel que par exemple une séparation en longueur d'onde ou encore un filtrage optique ou une polarisation du rayonnement optique.

On notera que cette problématique de dispositif de mesure optique non adapté est particulièrement présente lorsque le système optique comporte des lentilles en tant que composants optiques.

On note que par « rayonnement optique » il est entendu, ci-dessus et dans le reste de ce document, un rayonnement électromagnétique dont la ou les longueurs d'onde sont comprises dans la plage de longueurs d'onde du visible, c'est-à-dire entre 380 et 780 nm.

Ainsi, un dispositif apte à fonctionner dans un milieu ionisant doit être équipé d'un système de détection et d'un système optique aptes à fonctionner sous rayonnement ionisant.

Or, si les systèmes de détection optique adaptés pour les milieux ionisants sont relativement bien maitrisés, les inventeurs ont constaté un certain nombre d'inconvénients concernant les systèmes optiques polychromatiques aptes à fonctionner dans de tels milieux.

En effet, afin de limiter les aberrations chromatiques dans le visible, un système optique polychromatique doit comporter au moins une première partie de ses lentilles réalisée dans au moins un verre crown et au moins une deuxième partie de ses lentilles réalisée dans au moins un verre flint, ceci afin de former des doublets achromatiques. Ainsi, pour qu'un système optique soit apte à fonctionner sous rayonnement ionisant, chacun des verres dans lesquels sont formés ces composants optiques, et donc notamment ceux mentionnés ci-dessus formant les lentilles, doit également être adapté et donc présenter des caractéristiques de transmission peu, voire pas, dégradées par les rayonnements ionisants.

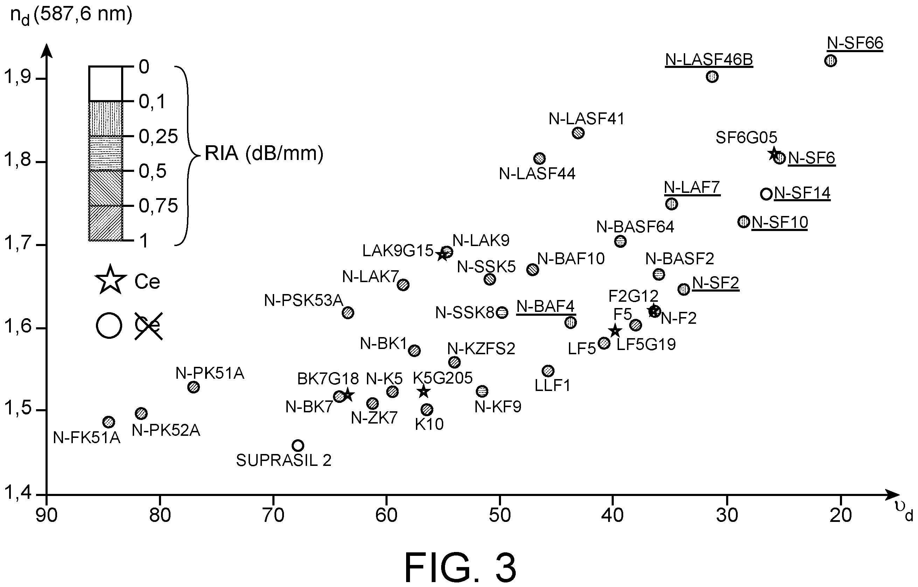

Par verre crown et verre flint il est entendu, ci-dessus et dans le reste de ce document, en conformité avec les connaissances générales de l'homme du métier, un verre qui présente un nombre d'Abbe $v_d$ qui est respectivement supérieur à 50 et inférieur à 50. On notera que, dans le présent document, comme cela sera discuté en lien avec la figure 3, le nombre d'Abbe $v_d$ est considéré relativement à la raie d de l'hélium à 587,5618 nm, le nombre d'Abbe étant donc déterminé à partir l'équation suivante :

$$v_d = \frac{n_d - 1}{n_F^2 - n_C}$$

Avec $n_d$ l'indice optique du verre considéré dans la raie d de l'hélium à 587,5618 nm, $n_F$ l'indice optique du verre considéré dans la raie F de l'hydrogène à 486,1 nm et $n_C$ l'indice optique du verre considéré dans la raie C de l'hydrogène à 656,3 nm. Bien entendu, l'enseignement du présent document peut aisément être extrapolé par l'homme du métier dans le cas où le nombre d'Abbe est déterminé à partir d'autres longueurs d'onde de référence, telles que la raie du sodium à 589,3 nm et la raie e du mercure à 546,073 nm.

En ce qui concerne les composants optiques, notamment les lentilles, en verre crown, il est connu d'utiliser de la silice pure, telle que le quartz, qui présente l'avantage d'être particulièrement résistant aux rayonnements ionisants.

En ce qui concerne les composants optiques, notamment les lentilles, en verre flint, il est connu, notamment des travaux de McGrath et de ses coauteurs publiés en 1976 dans la revue scientifique « Nuclear instruments and methods » n °135 pages 93 à 97, d'utiliser des verres dopés au cérium qui présentent une transmission qui est peu affectée par les rayonnements ionisants. Selon ces mêmes travaux, on notera qu'il est également possible d'utiliser des verres crown dopés au cérium en variante aux verres de silice pure.

On notera que pour l'homme du métier, outre la silice pure et les verres dopés au cérium et conformément notamment aux travaux de McGrath et de ses coauteurs, il n'existe pas d'autre verre, qu'il soit du type verre flint ou du type verre crown, qui puisse être adapté pour participer à la formation un système optique polychromatique apte à fonctionner en milieu ionisant.

Un tel système optique polychromatique apte à fonctionner dans un milieu ionisant, conformément à l'art antérieur, comprend donc nécessairement un verre dopé au cérium en tant que verre flint.

Or, les verres dopés au cérium ont un inconvénient majeur, outre leur coût relativement important vis-à-vis d'autres verres, qui est de présenter une réduction significative de transparence pour les rayonnements de longueur d'onde inférieure à 450 nm, c'est-à-dire dans bleu, avant toute irradiation. Ainsi, pour l'homme du métier, il n'est pas possible d'obtenir un dispositif de mesure optique qui présente à la fois une adaptation aux milieux ionisants et qui autorise une mesure optique fiable sur la quasi-totalité de la plage de longueurs d'onde du visible. Le document CN105676451 montre un dispositif de mesure optique avec sélection de verres flint résistant aux rayonnements ionisants.

## Exposé de l'invention

Les inventeurs se sont donnés comme but de développer un dispositif de mesure optique polychromatique qui soit apte à fonctionner en milieu ionisant et qui autorise une mesure optique fiable sur la totalité de la plage de longueurs d'onde du visible.

L'invention concerne à cet effet un procédé de fabrication d'un dispositif de mesure optique polychromatique apte à fonctionner sous rayonnement ionisant, le procédé comprenant les étapes suivantes :

- sélection d'au moins un premier verre d'optique crown dans un groupe de verres d'optique crown présentant chacun une composition exempte de cérium, ledit verre d'optique crown sélectionné étant dit verre crown,
- sélection d'au moins un premier verre d'optique flint dans un groupe de verres d'optique flint présentant chacun une composition exempte de cérium, ledit verre d'optique flint sélectionné étant dit verre flint,
- fourniture d'un système de détection apte à fonctionner sous rayonnement ionisant,
- formation d'au moins une première lentille en verre crown et d'au moins une deuxième lentille en verre flint,
- association d'au moins la première lentille et la deuxième lentille pour former un système optique du dispositif de mesure optique,
- association du système de détection et du système optique pour former le dispositif de mesure optique,

dans lequel l'étape de sélection du verre flint comprend les sous-étapes suivantes :

- mesure d'au moins une première grandeur représentative d'une transmission optique dans au moins une première plage de longueurs d'onde d'intérêt pour un échantillon de chaque verre du groupe de verres d'optique flint, ladite première grandeur représentative d'une transmission optique étant dite grandeur de transmission pré-irradiation,
- irradiation de chaque échantillon par un rayonnement ionisant avec une dose supérieure ou égale à 100 kGy,
- mesure d'au moins une deuxième grandeur représentative d'une transmission optique dans la première plage de longueurs d'onde d'intérêt pour chaque échantillon, ladite deuxième grandeur représentative d'une transmission optique étant dite grandeur de transmission post-irradiation,
- sélection du verre flint parmi les verres du groupe de verres d'optique flint pour lesquels une valeur moyenne de l'atténuation induite par irradiation dans la première plage de longueurs d'onde est inférieure à 0.25 dB/mm, ladite valeur moyenne de l'atténuation induite par irradiation étant déterminée à partir de la première grandeur représentative d'une transmission optique correspondante et de la deuxième grandeur représentative d'une transmission optique correspondante.

Avec un tel procédé il est possible, ceci à l'encontre des connaissances de l'homme du métier et des préjugés qui en découlent, de fabriquer un dispositif de mesure optique qui soit apte à fonctionner sous rayonnement ionisant en absence de composant optique en verre flint comprenant du cérium. Ainsi, une telle possibilité permet d'éviter les inconvénients de l'art antérieur qui utilise nécessairement un système optique comprenant des composants optiques en verre flint comprenant du cérium et ne permet donc pas de mesures optiques fiables sur la totalité de la plage de longueurs d'onde du visible, les verres comprenant du cérium présentant une absorbance dans les longueurs d'onde du bleu.

La valeur moyenne de l'atténuation induite par irradiation, RIA, peut être calculée à partir de l'équation suivante :

$$\overline{RIA} = -\frac{10}{L(\lambda2-\lambda1)} \int_{\lambda1}^{\lambda2} \log_{10}\left(\frac{I_{post}(\lambda)}{I_{pre}(\lambda)}\right) d\lambda$$

avec L une épaisseur de l'échantillon en millimètre, $I_{post}(\lambda)$ et $I_{pre}(\lambda)$ correspondant à une intensité de signal transmis correspondant respectivement à la grandeur de transmission post-irradiation et à la grandeur de transmission pré-irradiation à la longueur d'onde $\lambda$ et $\lambda1$ et $\lambda2$ étant respectivement la longueur d'onde de la borne minimale et maximale de la première plage de longueur d'onde.

La première plage de longueurs d'onde prédéterminée est comprise entre 350 nm et 600 nm, avantageusement entre 400 nm et 550 nm et, préférentiellement, entre 425 nm et 500 nm.

De telles plages permettent un choix particulièrement adapté du verre flint puisque c'est dans cette plage de longueurs d'onde que l'influence de l'irradiation sur la transmission est la plus forte.

Lors de la sous-étape d'irradiation de chaque échantillon par un rayonnement ionisant, le rayonnement ionisant est un rayonnement X ou gamma.

La grandeur de transmission pré-irradiation et la grandeur de transmission post-irradiation peuvent être sélectionnées dans le groupe comprenant une intensité d'un signal optique transmis, un taux ou un coefficient de transmission et un taux ou un coefficient d'absorption.

Le verre flint peut être sélectionné parmi le groupe de verres flint constitué par les références de verres N-SF2, N-SF6, N-SF10, N-SF14, N-SF66, N-LAF7, N-LASF46B et N-BAF4, le verre flint étant de préférence la référence de verre N-SF14, ces références de verres étant conformes à la norme SCHOTT.

Les inventeurs ont identifié, à partir du procédé selon l'invention, que ces verres présentent une RIA adaptée pour permettre la fabrication d'un système optique d'un dispositif de mesure optique apte à fonctionner sous rayonnement ionisant.

Lors de l'étape de sélection du verre flint il est sélectionné au moins un deuxième verre optique flint dit deuxième verre flint, ledit verre flint étant un autre verre flint que le verre flint parmi les verres du groupe de verres d'optique flint pour lesquels la valeur moyenne de l'atténuation induite par irradiation, RIA, dans la première plage de longueurs est inférieure à 0.25 dB/mm,

dans lequel lors de l'étape de formation d'au moins un premier composant optique en verre crown et d'au moins un deuxième composant optique en verre flint, il est également formé au moins un troisième composant optique en deuxième verre flint, et

dans lequel lors de l'étape d'association du premier composant optique et du deuxième composant optique pour former un système optique du dispositif de mesure optique, les premier composant optique et deuxième composant optique sont également associés au troisième composant optique pour former le système de mesure optique.

Ainsi, avec un tel deuxième verre et troisième composant optique avec lequel il est formé, il est possible, en combinaison avec le premier verre, et le verre crown, de précisément contrôler la réponse chromatique du dispositif de mesure optique polychromatique.

Lors de l'étape de sélection du verre crown, le verre crown sélectionné peut être un verre de silice pure.

L'invention concerne un dispositif de mesure optique apte à fonctionner sous rayonnement ionisant, ledit dispositif comprenant :

- un système de détection apte à fonctionner sous rayonnement ionisant,
- un système optique associé au système de détection, le système optique comprenant :

  • au moins un premier composant optique en verre en verre d'optique crown, ledit verre d'optique crown étant dit verre crown, et

- au moins un deuxième composant optique en un verre d'optique flint, dit verre flint,

dans lequel système optique les premier et deuxième composants optiques sont associés pour former ledit système optique.

Le verre flint est compris dans un groupe de verres d'optique flint présentant chacun une composition exempte de cérium,
et en ce que le verre flint présente une valeur moyenne de l'atténuation induite par irradiation dans la première plage de longueurs d'onde inférieure à 0.25 dB/mm, ladite valeur moyenne de l'atténuation induite par irradiation étant déterminée à partir d'une première grandeur représentative d'une transmission optique dudit verre flint et d'une deuxième grandeur représentative d'une transmission optique dudit verre flint.

La première grandeur représentative d'une transmission optique correspondant à une grandeur de transmission pré-irradiation dans au moins une première plage de longueurs d'onde d'intérêt pour un échantillon du verre flint, la deuxième grandeur représentative d'une transmission optique correspondant à une grandeur de transmission post-irradiation pour l'échantillon du verre flint obtenu après irradiation de l'échantillon de verre flint par un rayonnement ionisant avec une dose supérieure ou égale à 100 kGy.

Un tel dispositif est apte à fonctionner en milieu ionisant et qui autorise une mesure optique fiable sur la totalité de la plage de longueurs d'onde du visible.

La première plage de longueurs d'onde prédéterminée peut être comprise entre 350 nm et 600 nm, avantageusement entre 400 nm et 550 nm et, préférentiellement, entre 425 nm et 500 nm.

De telles plages permettent un choix particulièrement adapté du verre flint puisque c'est dans cette plage de longueurs d'onde que l'influence de l'irradiation sur la transmission est la plus forte.

Le verre flint peut être sélectionné parmi le groupe de verres flint constitué par les références de verres N-SF2, N-SF6, SF10, N-SF14, N-SF66, N-LAF7, N-LASF46B et N-BAF4, le verre flint étant de préférence la référence de verre N-SF14, ces références de verres étant conformes à la norme SCHOTT.

Le système optique peut comprend en outre au moins un troisième composant réalisé dans un deuxième verre optique flint dit deuxième verre flint optique flint autre que le verre flint, le deuxième verre flint étant compris dans le groupe de verres d'optique flint présentant chacun une composition exempte de cérium,
et le deuxième verre flint peut présenter la valeur moyenne de l'atténuation induite par irradiation, RIA, dans la première plage de longueurs inférieure à 0.25 dB/mm, ladite valeur moyenne de l'atténuation induite par irradiation, RIA.

Le verre crown peut être un verre de silice pure.

## Brève description des dessins

La présente invention sera mieux comprise à la lecture de la description d'exemples de réalisation, donnés à titre purement indicatif et nullement limitatif, en faisant référence aux dessins annexés sur lesquels :

La figure 1 illustre schématiquement un dispositif de mesure optique selon l'invention,
La figure 2 illustre schématiquement un banc de test permettant la mise en œuvre d'un procédé de fabrication d'un dispositif de mesure optique selon l'invention,
La figure 3 illustre graphiquement une étape de sélection de verre dans le cadre de la mise en œuvre d'un procédé de fabrication selon l'invention,
La figure 4 illustre graphiquement la comparaison du taux d'absorption d'un verre dopé au cérium avec un verre sélectionné dans le cadre d'un procédé de fabrication selon l'invention.

Des parties identiques, similaires ou équivalentes des différentes figures portent les mêmes références numériques de façon à faciliter le passage d'une figure à l'autre.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

## Description des modes de réalisation

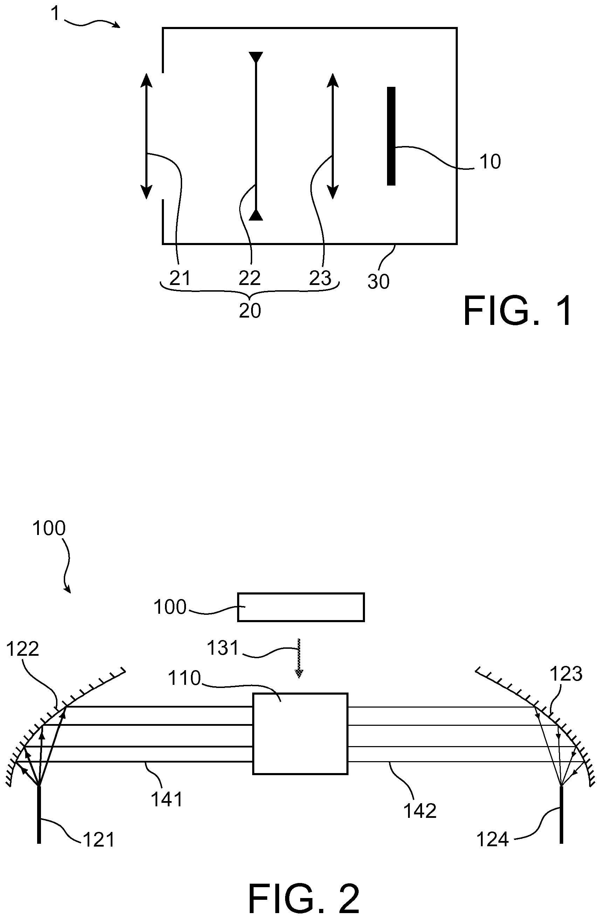

La figure 1 illustre schématiquement et à titre d'illustration un dispositif de mesure optique 1, tel qu'une caméra, ou encore un spectromètre, selon un premier mode de réalisation de l'invention, un tel dispositif de mesure optique 1 étant apte à fonctionner un milieu ionisant.

Comme illustré sur la figure 1, un tel dispositif de mesure optique 1 comporte :

- un système de mesure 10 apte à fonctionner sous rayonnements ionisants,
- un système optique 20 également apte à fonctionner sous rayonnements ionisants,
- un boîtier 30 enfermant au moins partiellement le système de mesure 10 et le système optique 20.

Les systèmes de mesure 10, tels que les capteurs photosensibles, aptes à fonctionner sous rayonnements ionisants sont des systèmes de mesure 10 qui présentent une sensibilité qui varie peu, voire pas, lorsque ces systèmes de mesure 10 sont soumis à un rayonnement ionisant. Pour plus d'information sur de tels systèmes de mesure, il est renvoyé aux travaux de Vincent Goiffon et de ses co-auteurs publiés dans le cadre de la conférence internationale « International Image Sensor Workshop (IISW 2015) » qui a eu lieu du 8 Juin 2015 au 11 Juin 2015 à Vaals aux Pays-Bas et dont le titre est « Toward Multi-MGy / Grad Radiation Hardened CMOS Image Sensors for Nuclear Applications *».* Il est également renvoyé aux travaux de Vincent Goiffron et de ses coauteurs publiés dans le journal scientifique « IEEE Transactions on nuclear science » volume 62 numéro 6 pages 2956 à 2964 en décembre 2015. Ainsi, de tels systèmes de mesure étant connus de l'art antérieur, ils ne sont pas décrits plus en avant dans le présent document.

Le système optique 20 comprend une pluralité de composants optiques 21, 22, 23 dont trois sont schématisés à titre d'exemple sur la figure 1.

Bien entendu, si sur l'exemple montré sur la figure 1 les composants optiques 21, 22, 23 sont tous des lentilles, dont deux lentilles 21, 23 sont des lentilles convergentes et une lentille 22 est une lentille divergente, un système optique selon l'invention peut comporter d'autres types de composants optiques que des lentilles, tels que des prismes ou encore des miroirs, étant entendu, que le système optique 20 comporte au moins une première et une deuxième lentille.

De la même façon, la formule optique du système optique 20 montrée sur la figure 1 n'est fournie qu'à titre d'exemple et d'illustration de l'invention. Ainsi, cette formule optique n'est nullement fonctionnelle, l'homme du métier, étant à même, à partir de ses connaissances générales et des règles de fabrication explicitées dans le présent document, de développer un système optique conforme au besoin du dispositif de mesure selon l'invention.

Selon une possibilité particulièrement avantageuse de l'invention, au moins l'un des composants optiques parmi les composants optiques 21, 22, 23 du système optique 20 est réalisé dans un verre de silice pure en tant que verre d'optique crown, ledit verre de silice pure étant dit verre crown.

En variante, au moins l'un des composants optiques parmi les composants optiques 21, 22, 23 du système optique 20 est réalisé dans un premier verre d'optique crown sélectionné dans un groupe de verres d'optique crown présentant chacun une composition exempte de cérium et qui est distinct de la silice pure, ledit verre d'optique crown sélectionné étant dit verre crown.

On notera que selon cette variante, un tel verre d'optique crown peut être sélectionné selon un principe similaire à celui mis en œuvre dans le cadre de la sélection d'un verre optique flint décrit ci-après.

Ainsi, au moins un autre des composants optiques parmi les composants optiques 21, 22, 23 du système optique 20 est réalisé dans un verre d'optique flint dans un groupe de verres d'optique flint, ledit verre d'optique flint étant dit verre flint.

Afin de choisir ce verre flint et ainsi fournir un dispositif de mesure optique 10 qui soit à la fois apte à fonctionner en milieu ionisant, les inventeurs ont mis en place un procédé de sélection du verre flint ceci en sélectionnant, à l'encontre des préjugés de l'homme du métier, des verres flint exempts de cérium.

Un tel procédé de sélection du verre flint comprend les étapes suivantes :

- sélection d'un groupe de verres flint exempt de cérium,
- mesure d'une première grandeur représentative d'une transmission optique dans au moins une première plage de longueurs d'onde d'intérêt pour un échantillon de chaque verre du groupe de verres d'optique flint, ladite première grandeur représentative d'une transmission optique étant dite grandeur de transmission pré-irradiation,
- irradiation de chaque échantillon par un rayonnement ionisant avec une dose supérieure ou égale à 100 kGy,
- mesure d'au moins une deuxième grandeur représentative d'une transmission optique dans la première plage de longueurs d'onde d'intérêt pour chaque échantillon, ladite deuxième grandeur représentative d'une transmission optique étant dite grandeur de transmission post-irradiation,
- sélection du verre flint parmi les verres du groupe de verres d'optique flint pour lesquels une valeur moyenne de l'atténuation induite par irradiation, RIA, dans la première plage de longueurs d'onde est inférieure à 0.25 dB/mm, ladite valeur moyenne de l'atténuation induite par irradiation étant déterminée à partir de la première grandeur représentative d'une transmission optique correspondante et de la deuxième grandeur représentative d'une transmission optique correspondante.

Plus précisément, afin de déterminer si l'inégalité ci-dessus est respectée, les inventeurs ont choisi comme première et deuxième grandeur représentatives d'une transmission optique respectivement une première et une deuxième intensité de signal transmise $I_{pre}$, $I_{post}$ pour un éclairage donné de l'échantillon de verre, ceci pour chacune des longueurs d'onde de la première plage de longueurs d'onde.

La première plage de longueurs d'onde prédéterminée peut être comprise entre 350 nm et 600 nm, avantageusement entre 400 nm et 550 nm et, préférentiellement, entre 425 nm et 500 nm. Pour la suite de ce document, c'est cette dernière plage de grandeur allant de 425 nm à 500 nm qui a été retenue.

Ainsi, à partir de cette première et deuxième intensité, les inventeurs sont à même de calculer une atténuation induite sous irradiation, ou RIA pour « Radiation Induced Attenuation », pour chacune des longueurs d'onde $\lambda$ comprises dans la première plage de longueurs d'onde prédéterminée, c'est-à-dire entre 425 nm et 500 nm sur la base du calcul suivant :

$$RIA\,(\lambda)=-\frac{10}{L}\log_{10}\left(\frac{I_{post}(\lambda)}{I_{pre}(\lambda)}\right)$$

avec L une épaisseur de l'échantillon en millimètre et $I_{post}(\lambda)$ et $I_{pre}(\lambda)$ correspondant respectivement à la première et à la deuxième intensité à la longueur d'onde $\lambda$. En appliquant ce calcul entre 425 nm et 500 nm, c'est-à-dire la première plage de longueurs d'onde et en faisant une moyenne des valeurs de RIA obtenue, la valeur moyenne de la RIA est alors connue et il est possible d'établir si l'inégalité précitée est respecté en vérifiant si cette valeur moyenne de RIA est inférieure ou égale à 0,25 dB/mm.

On notera qu'ainsi et en conformité avec l'équation ci-dessus, le calcul de la valeur moyenne de l'atténuation induite par irradiation, RIA, peut être calculée à partir de l'équation suivante :

$$\overline{RIA}=-\frac{10}{L(\lambda2-\lambda1)}\int_{\lambda1}^{\lambda2}\log_{10}\left(\frac{I_{post}(\lambda)}{I_{pre}(\lambda)}\right)\,d\lambda$$

avec L une épaisseur de l'échantillon en millimètre, $I_{post}(\lambda)$ et $I_{pre}(\lambda)$ correspondant à une intensité de signal transmis correspondant respectivement à la grandeur de transmission post-irradiation et à la grandeur de transmission pré-irradiation à la longueur d'onde $\lambda$, et $\lambda1$ et $\lambda2$ étant respectivement la longueur d'onde de la borne minimale et maximale de la première plage de longueur d'onde.

Bien entendu, ce choix par les inventeurs, dicté par des raisons de praticité, n'est nullement limitatif de l'invention. Un homme du métier est en effet, parfaitement à même, sur la base du présent enseignement, de déterminer si cette inégalité est respectée à partir d'autres grandeurs représentatives d'une transmission optique, telle que par exemple une absorption ou encore un taux de transmission.

Pour mettre en œuvre un tel procédé de sélection de verre, les inventeurs ont développé un premier système de test 100 pour tester des échantillons de verre 110 dont un schéma de principe est figuré sur la figure 2.

Un tel système de test 100 comprend :

- une source de lumière 121 adaptée pour émettre un rayonnement 141 dans la première plage de longueurs d'onde d'intérêt avec une intensité prédéterminée,
- un premier système de transmission optique 122, ici un miroir parabolique, agencé pour transmettre le rayonnement émis par la source de lumière en direction de l'échantillon de verre 110 à tester,
- un deuxième système de transmission optique 123, ici également un miroir parabolique, agencé pour transmettre la partie rayonnement non absorbée 142 sortant de l'échantillon de verre 110 à tester,
- un détecteur optique agencé pour recevoir la partie de rayonnement non absorbée transmis par le deuxième système de transmission optique 142 et adapté pour mesurer l'intensité de ladite partie de rayonnement non absorbée,
- une source de rayonnement ionisant, ici une source de rayon X, agencée et adaptée pour soumettre l'échantillon de verre à une dose de rayonnement ionisant 141 supérieure à 100 kGy.

On notera que pour le système de test, les inventeurs ont plus précisément choisi la configuration suivante:

- une source de lumière blanche, celle-ci étant la source commercialisée par la société Ocean Optics™ sous la référence DH2000, en tant que source de lumière 121, le rayonnement émis par cette source étant transmis au premier système de transmission optique 122 au moyen d'une fibre optique de déport multimode, avantageusement résistante aux radiations, qui est la seule illustrée sur la figure 2,
- un premier et un deuxième système de transmission optique 122, 123 fournis chacun par un miroir parabolique commercialisé par la société Thorlabs sous la référence RC02SMA-F01, ceci de manière à fournir respectivement un rayonnement 141 parallèle en direction de l'échantillon de verre à tester 110 et à focaliser la partie du rayonnement non absorbée 142 pour une injection dans une fibre optique multimode,

- un spectrophotomètre en tant que détecteur optique, celui-ci étant le spectrophotomètre commercialisé par la société Ocean Optics™ sous la référence HR4000, celui-ci étant équipé d'une fibre optique multimode dans laquelle est injectée la partie du rayonnement non absorbée 142 par le deuxième système de transmission optique,
- un tube à rayon X opérant à 100 kV et adapté pour délivrer des photons X présentant une énergie moyenne de 40 eV.

Un tel système permet de suivre tout au long de l'irradiation la variation de la transmission de l'échantillon de verre et d'ainsi remonter à l'absorption induite dans l'échantillon de verre en fonction de l'irradiation.

En variante, les inventeurs ont également utilisé un système de mesure de spectrométrie du commerce pour mesurer la transmittance avant et post irradiation, l'échantillon de verre étant alors soumis à un rayonnement ionisant indépendamment du système de mesure.

Selon cette variante, les inventeurs ont plus précisément utilisé un système de mesure de spectrométrie dans le visible développé par Agilent™ et commercialisé sous la référence Cary-5000-UV-Vis-NIR. Dans le cadre de l'irradiation, dans une telle configuration, les inventeurs ont utilisés une source gamma pour irradier les échantillons de verre.

C'est dans le cadre d'un tel procédé que les inventeurs ont testé des verres exempts de cérium et, à titre de comparaison, certains verres comprenant un dopage au cérium, ceci pour une première plage de longueurs d'onde d'intérêt allant de 425 à 500 nm et à partir du système de mesure de spectrométrie selon la variante explicitée ci-dessus. On notera que pour ce faire, chacun des échantillons a été soumis à une irradiation gamma de 1 MGy (en dose équivalente $SiO_2$). En effet, cette plage de longueurs d'onde correspond aux longueurs d'onde du bleu pour lesquelles les verres dopés au cérium sont connus pour présenter une atténuation intrinsèque.

Bien entendu, ce choix de plage de longueurs d'onde fait par les inventeurs, s'il a été fait afin de montrer l'avantage de l'invention vis-à-vis des verres flint qu'aurait retenus l'homme du métier à partir de ses connaissances générales, n'est qu'indicatif.

Les inventeurs ont ainsi testé les verres suivants qui sont exempts de cérium : N-FK51, N-PK52A, N-PK51A, SUPRASIL 2, N-BK7, N-PSK53A, N-ZK7, N-K5, K10, N-LAK7, N-BK1, N-LAK9, N-KZFS2, N-KF9, N-SSK8, N-SSK5, LLF1, N-BAF4, N-BAF10, N-LASF41, N-LASF44, LF5, F5, LF5G19, N-F2, N-BASF2, N-BASF64, N-LAF7, N-LASF46B, N-SF10, N-SF14, N-SF6 et N-SF66, les références utilisées étant conformes à la norme de la société SCHOTT, dite par la suite norme SCHOTT. Comme indiqué ci-dessus, Ils ont également testé, à titre de comparaison, les verres suivants qui comprennent du cérium en tant que dopant : BK7G18, F2G12, LF5G19, K5G20, LAK9G15 et SF6G05, les références utilisées étant également conformes à la norme SCHOTT.

On notera que si dans le présent document, il a été décidé d'utiliser la norme SCHOTT, l'homme du métier est en mesure d'identifier les verres correspondants notamment en ce qui concerne les normes des sociétés OHARA, HOYA et CDGM à partir de documents identifiant les équivalences, par exemple à partir du document fourni par la société ESCO optic au lien internet suivant https://cdn.shopify.com/s/files/1/1090/0622/files/Glass Reference Chart-2.pdf (lien consulté le 3 avril 2019).

Ainsi, parmi ces verres exempts de cérium, on pourra notamment citer le verre N-SF14 pour lequel les inventeurs ont mesuré une valeur RIA moyenne de 0.09 dB/mm, ce qui est particulièrement faible et parfaitement inattendue pour l'homme du métier qui se serait attendu, pour un tel verre non dopé au cérium, à une atténuation beaucoup plus importante et donc à une RIA plus importante. On notera que, de plus, contrairement à ce à quoi s'attend l'homme du métier, certain verres comprenant du cérium en tant qu'élément dopant, tels que le verre SF6G05, présentent une valeur de RIA particulièrement forte, les inventeurs ayant mesuré une valeur moyenne supérieure à 0,75 dB/mm pour le verre SF6G05.

La figure 3 permet d'illustrer les résultats obtenus par les inventeurs lors de la mise en œuvre du procédé de sélection d'un verre flint. Cette figure représente l'ensemble des verres testés avec, en abscisses, le nombre d'Abbe et, en ordonnées, l'indice de réfraction dudit verre pour la raie d de l'hélium à 587,5618 nm, la valeur RIA étant figurée par une échelle de gris allant de 0 à 1. De même, on notera de plus que, en plus de leur référence SCHOTT du verre indiqué à côté du point de mesure correspondant, les verres exempts de cérium sont figurés au moyen de ronds et que les verres dopés au cérium sont figurés au moyen d'étoiles à 5 branches.

On peut ainsi voir sur la figure 3 que, contre toute attente, les inventeurs, par la mise en œuvre du procédé de sélection du verre flint qu'ils ont développé, ont sélectionné un certain nombre de verres non dopés au cérium qui sont adaptés pour réaliser une mesure optique en milieu ionisant puisqu'ils présentent une valeur moyenne de RIA inférieure ou égale à 0,25 dB/mm dans la première plage de longueurs d'onde. Ces verres exempts de cérium ont été surlignés sur la figure 3 afin de les mettre en évidence.

Ces verres identifiés par les inventeurs sont donc le verre N-SF2 qui présente une RIA moyenne de 0,2 dB/mm, le verre N-SF6 qui présente une RIA moyenne de 0, 11 dB/mm, le verre N-SF10 qui présente une RIA moyenne de 0,21 dB/mm, le verre N-SF14 qui présente une RIA moyenne de 0,09 dB/mm, le verre N-SF66 qui présente une RIA moyenne de 0,24 dB/mm, le verre N-LAF7 qui présente une RIA moyenne de 0,18 dB/mm, le N-LASF46B qui présente une RIA moyenne de 0,2 dB/mm et le verre N-BAF4 qui présente une RIA moyenne de 0,2 dB/mm.

Ainsi, à partir du procédé d'identification de verre flint mis en œuvre par les inventeurs, ceux-ci ont pu identifier un verre flint adapté pour la fabrication d'un système optique pour un dispositif de mesure optique 1 conforme à l'invention. Selon cette identification, un dispositif de mesure optique 1 selon l'invention peut donc comporter, pour son système optique 20, une première partie de ses composants optiques 21 réalisés dans du verre de silice en tant que verre crown et une deuxième partie de ses composants optiques 22 réalisés dans un verre optique flint sélectionné parmi le groupe de verres flint constitué par les références de verres N-SF2, N-SF6, N-SF10, N-SF14, N-SF66, N-LAF7, N-LASF46B et N-BAF4, le verre flint étant de préférence la référence de verre N-SF14.

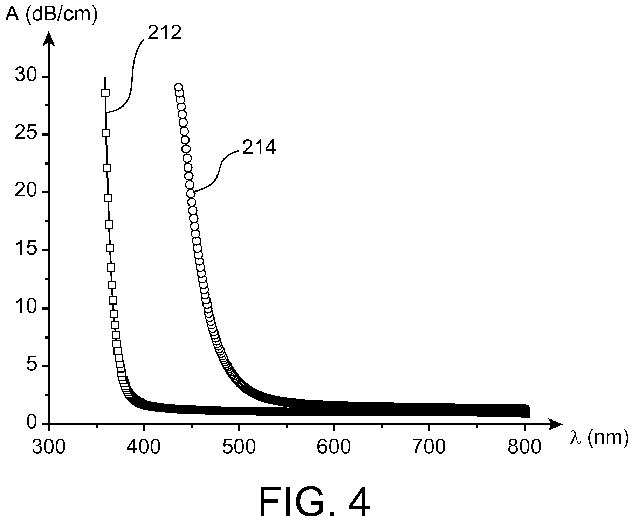

La figure 4 illustre ainsi l'avantage d'un choix de verre flint selon le procédé de l'invention en comparant le taux d'absorption d'un verre flint sélectionné dans le groupe ci-dessus, ce verre étant le verre N-SF6 vis-à-vis d'un verre flint dopé au cérium qui aurait été sélectionné par l'homme du métier conformément à ces connaissances générales, ce verre étant le verre SF6G05, ces deux verres ayant été soumis à une dose identique de 100 kGy (en dose équivalente $SiO_2$).

On peut ainsi voir sur la figure 4, qui montre le taux d'absorption A en dB par centimètre en fonction de la longueur d'onde $\lambda$, que le taux d'absorption 211 du verre flint dopé au cérium augmente drastiquement en dessous de 500 nm, le verre flint dopé au cérium devenant quasi opaque en dessous de 450 nm. En ce qui concerne le verre sélectionné selon le procédé de l'invention, le taux d'absorption ne commence à croître qu'en dessous de 380 nm assurant ainsi une bonne transmission sur quasiment toute la plage de longueurs d'onde du visible.

Dans un dispositif selon l'invention, conformément à la figure 1, le système de mesure 10 et le système optique 20 peuvent être associés au moyen du boîtier 30.

Partant du procédé de sélection du verre flint explicité ci-dessus, un procédé de fabrication d'un dispositif de mesure 1 selon l'invention peut comporter les étapes suivantes :

- sélection d'au moins un premier verre d'optique crown dans un groupe de verres d'optique crown présentant chacun une composition exempte de cérium, ledit verre d'optique crown sélectionné étant dit verre crown, le verre crown étant préférentiellement un verre de silicepure,
- sélection d'au moins un premier verre d'optique flint dans le groupe de verres d'optique flint présentant chacun une composition exempte de cérium, ledit premier verre d'optique flint sélectionné étant dit verre flint,
- fourniture du système de détection 10 apte à fonctionner sous rayonnement ionisant,
- formation d'une première partie de composants optiques 21 en verre crown et d'une deuxième partie de composants optiques 22 en verre flint,
- association de la première partie de composants optiques 21 et de la deuxième partie de deuxièmes composants optiques 22 pour former le système optique 20 du dispositif de mesure optique 1,
- association du système de détection 10 et du système optique 20 pour former le dispositif de mesure optique 1.

Dans ce procédé de fabrication, l'étape de sélection du premier verre d'optique flint est notamment conforme au procédé de sélection de l'invention et comprend ainsi les sous-étapes suivantes :

- mesure d'au moins une première grandeur représentative d'une transmission optique dans au moins une première plage de longueurs d'onde d'intérêt pour un échantillon 110 de chaque verre du groupe de verres d'optique flint, ladite première grandeur représentative d'une transmission optique étant dite grandeur de transmission pré-irradiation,
- irradiation de chaque échantillon 110 par un rayonnement ionisant avec une dose supérieure ou égale à 100 kGy (en dose équivalente $SiO_2$),
- mesure d'au moins une deuxième grandeur représentative d'une transmission optique dans la première plage de longueurs d'onde d'intérêt pour chaque échantillon 110, ladite deuxième grandeur représentative d'une transmission optique étant dite grandeur de transmission post-irradiation,
- sélection du verre flint parmi les verres du groupe de verres d'optique flint pour lesquels pour lesquels une valeur moyenne de l'atténuation induite par irradiation, RIA, dans la première plage de longueurs est inférieure à 0.25 dB/mm, ladite valeur moyenne de l'atténuation induite par irradiation étant déterminée à partir de la première grandeur représentative d'une transmission optique correspondante et de la deuxième grandeur représentative d'une transmission optique correspondante.

Comme déjà vue, la valeur moyenne de l'atténuation induite par irradiation, RIA, peut notamment être calculée à partir de l'équation suivante :

$$\overline{RIA} = -\frac{10}{L(\lambda2-\lambda1)} \int_{\lambda1}^{\lambda2} \log_{10}\left(\frac{I_{post}(\lambda)}{I_{pre}(\lambda)}\right) d\lambda$$

avec L une épaisseur de l'échantillon en millimètre, $I_{post}(\lambda)$ et $I_{pre}(\lambda)$ correspondant à une intensité de signal transmis correspondant respectivement à la grandeur de transmission post-irradiation et à la grandeur de transmission pré-irradiation à la longueur d'onde λ et λ1 et λ2 étant respectivement la longueur d'onde de la borne minimale et maximale de la première plage de longueur d'onde.

On notera que, avec un tel calcul, la première grandeur représentative d'une transmission optique dans au moins une première plage de longueurs d'onde d'intérêt, peut correspondre aux intensités de transmission pré-irradiation obtenues pour la première plage de longueurs d'onde. De même conformément à cette possibilité, la deuxième grandeur représentative d'une transmission optique dans au moins une première plage de longueurs d'onde d'intérêt, peut correspondre aux intensités de transmission post-irradiation obtenues pour la première plage de longueurs d'onde.

Selon un deuxième mode de réalisation de l'invention basé également sur la figure 2, le système optique 20 peut comporter une première partie de ses composants optiques 21 réalisée dans du verre de silice en tant que verre crown, une deuxième partie de ses composants optiques 22 réalisée dans un premier verre optique flint sélectionné parmi le groupe de verres flint exempt de cérium, ce verre étant dit verre flint, et une troisième partie de ses composants optiques 23 réalisée dans un deuxième verre optique flint sélectionné parmi le groupe de verres flint, dit deuxième verre flint, le deuxième verre flint étant différent du verre flint.

Ainsi, un dispositif de mesure optique 1 selon ce deuxième mode de réalisation se différencie d'un dispositif de mesure optique 1 selon le premier mode de réalisation en ce que son dispositif optique 20 comporte en outre une troisième partie de composants optiques 23 réalisée dans un deuxième verre optique flint sélectionné parmi le groupe de verres flint, dit deuxième verre flint, le deuxième verre flint étant différent du verre flint.

Conformément à ce deuxième mode de réalisation, le deuxième verre flint est sélectionné lors de l'étape de sélection du verre flint conformément au procédé de sélection développé par les inventeurs.

En effet, dans le cadre de cette sélection du verre flint, il peut être également sélectionné le deuxième verre flint, ledit deuxième verre flint étant un autre verre flint que le verre flint parmi les verres du groupe de verres d'optique flint pour lesquels lka valeur moyenne de l'atténuation induite par irradiation, RIA, dans la première plage de longueurs d'onde est inférieure à 0.25 dB/mm.

De même, un procédé de fabrication d'un dispositif de mesure optique selon ce deuxième mode de réalisation se différencie d'un procédé de fabrication d'un dispositif de mesure optique selon le premier mode de réalisation en ce que lors de l'étape de formation de la première partie de composants optiques 21 en verre crown et de la deuxième partie de composants optiques 22 en verre flint, il est également formé une troisième partie de composants optiques 23 en deuxième verre flint, et en ce que lors de l'étape d'association du premier composant optique 21 et du deuxième composant optique 22 pour former un système optique 2 du dispositif de mesure optique 1, les premier composant optique 21 et deuxième composant optique 22 sont également associés au troisième composant optique 23 pour former le système de mesure optique 20.

On notera, comme cela a déjà été indiqué plus haut, que l'étape de sélection d'au moins un premier verre d'optique crown peut être mise en œuvre d'une manière similaire à l'étape de sélection d'au moins un premier verre d'optique flint. Selon une telle possibilité, l'étape de sélection du verre crown peut comprendre les sous-étapes suivantes :

- mesure d'au moins une première grandeur représentative d'une transmission optique dans au moins une première plage de longueurs d'onde d'intérêt pour un échantillon de chaque verre du groupe de verres d'optique crown, ladite première grandeur représentative d'une transmission optique étant dite grandeur de transmission pré-irradiation,
- irradiation de chaque échantillon par un rayonnement ionisant avec une dose supérieure ou égale à 100 kGy,
- mesure d'au moins une deuxième grandeur représentative d'une transmission optique dans la première plage de longueurs d'onde d'intérêt pour chaque échantillon, ladite deuxième grandeur représentative d'une transmission optique étant dite grandeur de transmission post-irradiation,
- sélection du verre crown parmi les verres du groupe de verres d'optique crown pour lesquels une valeur moyenne de l'atténuation induite par irradiation, RIA, dans la première plage de longueurs est inférieure à 0.25 dB/mm, ladite valeur moyenne de l'atténuation induite par irradiation étant déterminée à partir de la première grandeur représentative d'une transmission optique correspondante et de la deuxième grandeur représentative d'une transmission optique correspondante.

**Revendications**

1.  Procédé de fabrication d'un dispositif de mesure optique (1) polychromatique apte à fonctionner sous rayonnement ionisant, le procédé comprenant les étapes suivantes :

    - sélection d'au moins un premier verre d'optique crown dans un groupe de verres d'optique crown présentant chacun une composition exempte de cérium, ledit verre d'optique crown sélectionné étant dit verre crown,
    - sélection d'au moins un premier verre d'optique flint dans un groupe de verres d'optique flint présentant chacun une composition exempte de cérium, ledit verre d'optique flint sélectionné étant dit verre flint,

- fourniture d'un système de détection (10) apte à fonctionner sous rayonnement ionisant,
- formation d'au moins un premier composant optique (21) en verre crown et d'au moins un deuxième composant optique (22) en verre flint,
- association du premier composant optique (21) et du deuxième composant optique (22) pour former un système optique (20) du dispositif de mesure optique (1),
- association du système de détection (10) et du système optique (20) pour former le dispositif de mesure optique (1),

dans lequel l'étape de sélection du verre flint comprend les sous-étapes suivantes :

- mesure d'au moins une première grandeur représentative d'une transmission optique dans au moins une première plage de longueurs d'onde d'intérêt pour un échantillon (110) de chaque verre du groupe de verres d'optique flint, ladite première grandeur représentative d'une transmission optique étant dite grandeur de transmission pré-irradiation,
- irradiation de chaque échantillon (110) par un rayonnement ionisant avec une dose supérieure ou égale à 100 kGy,
- mesure d'au moins une deuxième grandeur représentative d'une transmission optique dans la première plage de longueurs d'onde d'intérêt pour chaque échantillon (110), ladite deuxième grandeur représentative d'une transmission optique étant dite grandeur de transmission post-irradiation,
- sélection du verre flint parmi les verres du groupe de verres d'optique flint pour lesquels une valeur moyenne de l'atténuation induite par irradiation, RIA, dans la première plage de longueurs est inférieure à 0.25 dB/mm, ladite valeur moyenne de l'atténuation induite par irradiation étant déterminée à partir de la première grandeur représentative d'une transmission optique correspondante et de la deuxième grandeur représentative d'une transmission optique correspondante.

2. Procédé de fabrication d'un dispositif de mesure optique (1) polychromatique apte à fonctionner sous rayonnement ionisant selon la revendication 1, dans lequel la valeur moyenne de l'atténuation induite par irradiation, RIA, est calculée à partir de l'équation suivante :

$$\overline{RIA} = -\frac{10}{L(\lambda 2 - \lambda 1)} \int_{\lambda 1}^{\lambda 2} \log_{10}\left(\frac{I_{post}(\lambda)}{I_{pre}(\lambda)}\right) d\lambda$$

avec L une épaisseur de l'échantillon en millimètre, $I_{post}(\lambda)$ et $I_{pre}(\lambda)$ correspondant à une intensité de signal transmis correspondant respectivement à la grandeur de transmission post-irradiation et à la grandeur de transmission pré-irradiation à la longueur d'onde $\lambda$, et $\lambda 1$ et $\lambda 2$ étant respectivement la longueur d'onde de la borne minimale et maximale de la première plage de longueur d'onde.

3. Procédé de fabrication d'un dispositif de mesure optique (1) polychromatique apte à fonctionner sous rayonnement ionisant selon la revendication 1 ou 2, dans lequel la première plage de longueurs d'onde prédéterminée est comprise entre 350 nm et 600 nm, avantageusement entre 400 nm et 550 nm et, préférentiellement, entre 425 nm et 500 nm.

4. Procédé de fabrication d'un dispositif de mesure optique (1) polychromatique apte à fonctionner sous rayonnement ionisant selon les revendications 1 à 3, dans lequel lors de la sous-étape d'irradiation de chaque échantillon par un rayonnement ionisant, le rayonnement ionisant est rayonnement X ou gamma.

5. Procédé de fabrication d'un dispositif de mesure optique (1) apte à fonctionner sous rayonnement ionisant selon l'une quelconque des revendications 1 à 4, dans lequel la grandeur de transmission pré-irradiation et la grandeur de transmission post-irradiation sont sélectionnées dans le groupe comprenant une intensité d'un signal optique transmis, un taux ou un coefficient de transmission et un taux ou un coefficient d'absorption.

6. Procédé de fabrication d'un dispositif de mesure optique (1) polychromatique apte à fonctionner sous rayonnement ionisant selon l'une quelconque des revendications 1 à 5, dans lequel le verre flint est sélectionné parmi le groupe de verres flint constitué par les références de verres N-SF2, N-SF6, N-SF10, N-SF14, N-SF66, N-LAF7, N-LASF46B et N-BAF4, le verre flint étant de préférence la référence de verre N-SF14, ces références de verres étant conformes à la norme SCHOTT.

7. Procédé de fabrication d'un dispositif de mesure optique (1) polychromatique apte à fonctionner sous rayonnement ionisant selon l'une quelconque des revendications 1 à 6, dans lequel lors de l'étape de sélection du verre flint il est sélectionné au moins un deuxième verre optique flint dit deuxième verre flint, ledit verre flint étant un autre verre flint que le verre flint parmi les verres du groupe de verres d'optique flint pour lesquels la valeur moyenne de l'atténuation induite par irradiation, RIA, dans la première plage de longueurs d'onde est inférieure à 0.25 dB/mm,

dans lequel lors de l'étape de formation d'au moins un premier composant optique (21) en verre crown et d'au moins un deuxième composant optique (22) en verre flint, il est également formé au moins un troisième composant optique (23) en deuxième verre flint, et

dans lequel lors de l'étape d'association du premier composant optique (21) et du deuxième composant optique (22) pour former un système optique (20) du dispositif de mesure optique (1), les premier composant optique (21) et deuxième composant optique (22) sont également associé au troisième composant optique (23) pour former le système de mesure optique (20).

8. Procédé de fabrication d'un dispositif de mesure optique (1) polychromatique apte à fonctionner sous rayonnement ionisant selon l'une quelconque des revendications 1 à 7, dans lequel lors de l'étape de sélection du verre crown, le verre crown sélectionné est un verre de silice pure.

9. Dispositif de mesure optique (1) polychromatique apte à fonctionner sous rayonnement ionisant, ledit dispositif comprenant :

- un système de détection (10) apte à fonctionner sous rayonnement ionisant,
- un système optique (20) associé au système de détection (10), le système optique (20) comprenant :

• au moins un premier composant optique (21) en verre d'optique crown, ledit verre d'optique crown étant dit verre crown, et
• au moins un deuxième composant (22) optique en un verre d'optique flint, dit verre flint,

dans lequel système optique (20) les premier et deuxième composants optiques (21, 22) sont associés pour former ledit système optique (20),

le dispositif de mesure optique (20) **<u>étant caractérisé en ce que</u>** le verre flint est compris dans un groupe de verres d'optique flint présentant chacun une composition exempte de cérium,

le verre crown étant compris dans un groupe de verres d'optique crown présentant chacun une composition exempte de cérium,

et **en ce que** le verre flint présente une valeur moyenne de l'atténuation induite par irradiation dans la première plage de longueurs d'onde inférieure à 0.25 dB/mm, ladite valeur moyenne de l'atténuation induite par irradiation étant déterminée à partir d'au moins une première grandeur représentative d'une transmission optique dudit verre flint et d'au moins une deuxième grandeur représentative d'une transmission optique dudit verre flint,

la première grandeur représentative d'une transmission optique correspondant à une grandeur de transmission pré-irradiation dans au moins une première plage de longueurs d'onde d'intérêt pour un échantillon du verre flint,

la deuxième grandeur représentative d'une transmission optique correspondant à une grandeur de transmission post-irradiation pour l'échantillon du verre flint obtenu après irradiation de l'échantillon de verre flint par un rayonnement ionisant avec une dose supérieure ou égale à 100 kGy.

10. Dispositif de mesure optique (1) apte à fonctionner sous rayonnement ionisant selon la revendication 9, dans lequel la première plage de longueurs d'onde prédéterminée est comprise entre 350 nm et 600 nm, avantageusement entre 400 nm et 550 nm et, préférentiellement, entre 425 nm et 500 nm.

11. Dispositif de mesure optique (1) selon la revendication 9 ou 10, dans lequel le verre flint est sélectionné parmi le groupe de verres flint constitué par les références de verres N-SF2, N-SF6, N-SF10, N-SF14, N-SF66, N-LAF7, N-LASF46B et N-BAF4, le verre flint étant de préférence la référence de verre N-SF14, ces références de verres étant conformes à la norme SCHOTT.

12. Dispositif de mesure optique (1) selon l'une quelconque des revendications 9 à 11 dans lequel le système optique (20) comprend en outre au moins un troisième composant réalisé dans un deuxième verre optique flint dit deuxième verre flint optique flint autre que le verre flint, le deuxième verre flint étant compris dans le groupe de verres d'optique flint présentant chacun une composition exempte de cérium,
et le deuxième verre flint présente la valeur moyenne de l'atténuation induite par irradiation, RIA, dans la première plage de longueurs inférieure à 0.25 dB/mm, ladite valeur moyenne de l'atténuation induite par irradiation, RIA.

13. Dispositif de mesure optique (1) selon l'une quelconque des revendications 9 à 12, dans lequel le verre crown est un verre de silice pure.

**Patentansprüche**

1. Verfahren zur Herstellung einer polychromatischen optischen Messvorrichtung (1), die imstande ist, unter ionisierender Strahlung zu arbeiten, wobei das Verfahren die folgenden Schritte umfasst:

   - Auswählen mindestens eines ersten optischen Kronglases aus einer Gruppe optischer Krongläser, die jeweils eine Cer-freie Zusammensetzung aufweisen, wobei das ausgewählte optische Kronglas Kronglas genannt wird,
   - Auswählen mindestens eines ersten optischen Flintglases aus einer Gruppe von optischen Flintgläsern, die jeweils eine Cer-freie Zusammensetzung aufweisen, wobei das ausgewählte optische Flintglas Flintglas genannt wird,
   - Bereitstellen eines Erkennungssystems (10), das imstande ist, unter ionisierender Strahlung zu arbeiten,
   - Bilden mindestens einer ersten optischen Komponente (21) aus Kronglas und mindestens einer zweiten optischen Komponente (22) aus Flintglas,
   - Vereinigen der ersten optischen Komponente (21) und der zweiten optischen Komponente (22), um ein optisches System (20) der optischen Messvorrichtung (1) zu bilden,
   - Vereinigen des Erkennungssystems (10) und des optischen Systems (20), um die optische Messvorrichtung (1) zu bilden,

   wobei der Schritt zum Auswählen des Flintglases die folgenden Teilschritte umfasst:

   - Messen mindestens einer ersten repräsentativen Größe einer optischen Übertragung in mindestens einem ersten Wellenlängenbereich von Interesse für eine Probe (110) eines jeden Glases der Gruppe von optischen Flintgläsern, wobei die erste repräsentative Größe einer optischen Übertragung Vorbestrahlungsübertragungsgröße genannt wird,
   - Bestrahlen einer jeden Probe (110) durch eine ionisierende Strahlung mit einer Dosis größer oder gleich 100 kGy,
   - Messen mindestens einer zweiten repräsentativen Größe einer optischen Übertragung im ersten Wellenlängenbereich von Interesse für jede Probe (110), wobei die zweite repräsentative Größe einer optischen Übertragung Nachbestrahlungsübertragungsgröße genannt wird,
   - Auswählen des Flintglases aus der Gruppe von optischen Flintgläsern, bei denen ein Mittelwert der durch Bestrahlung induzierten Abschwächung, RIA, im ersten Längenbereich kleiner als 0,25 dB/mm ist, wobei der Mittelwert der durch Bestrahlung induzierten Abschwächung aus der ersten repräsentativen Größe einer entsprechenden optischen Übertragung und der zweiten repräsentativen Größe einer entsprechenden optischen Übertragung bestimmt wird.

2. Verfahren zur Herstellung einer polychromatischen optischen Messvorrichtung (1), die imstande ist, unter ionisierender Strahlung zu arbeiten, nach Anspruch 1, wobei der Mittelwert der durch Bestrahlung induzierten Abschwächung, RIA, aus der folgenden Gleichung berechnet wird:

$$\overline{RIA} = -\frac{10}{L(\lambda 2 - \lambda 1)} \int_{\lambda 1}^{\lambda 2} log_{10}\left(\frac{l_{post}(\lambda)}{l_{pre}(\lambda)}\right) d\lambda$$

wobei L eine Dicke der Probe in Millimetern ist, $l_{post}(\lambda)$ und , $l_{pre}(\lambda)$ einer Intensität eines übertragenen Signals entspricht, die jeweils der Nachbestrahlungsübertragungsgröße und der Vorbestrahlungsübertragungsgröße mit der Wellenlänge $\lambda$ entsprechend, und $\lambda 1$ und $\lambda 2$ jeweils die Wellenlänge der minimalen und maximalen Grenze des ersten Wellenlängenbereichs sind.

3. Verfahren zur Herstellung einer polychromatischen optischen Messvorrichtung (1), die imstande ist, unter ionisierender Strahlung zu arbeiten, nach Anspruch 1 oder 2, wobei der erste vorbestimmte Wellenlängenbereich zwischen 350 nm und 600 nm, vorteilhafterweise zwischen 400 nm und 550 nm und vorzugsweise zwischen 425 nm und 500 nm umfasst ist.

4. Verfahren zur Herstellung einer polychromatischen optischen Messvorrichtung (1), die imstande ist, unter ionisierender Strahlung zu arbeiten, nach den Ansprüchen 1 bis 3, wobei beim Teilschritt zum Bestrahlen einer jeden Probe durch eine ionisierende Strahlung die ionisierende Strahlung Röntgen- oder Gamma-Strahlung ist.

5. Verfahren zur Herstellung einer polychromatischen optischen Messvorrichtung (1), die imstande ist, unter ionisierender Strahlung zu arbeiten, nach einem der Ansprüche 1 bis 4, wobei die Vorbestrahlungsübertragungsgröße und die Nachbestrahlungsübertragungsgröße aus der Gruppe ausgewählt werden, die eine Intensität eines übertragenen optischen Signals, eine Übertragungsrate oder einen Übertragungskoeffizienten und eine Absorptionsrate oder einen Absorptionskoeffizienten umfasst.

6. Verfahren zur Herstellung einer polychromatischen optischen Messvorrichtung (1), die imstande ist, unter ionisierender Strahlung zu arbeiten, nach einem der Ansprüche 1 bis 5, wobei das Flintglas aus der Gruppe von Flintgläsern ausgewählt wird, die durch die Glasreferenzen N-SF2, N-SF6, N-SF10, N-SF14, N-SF66, N-LAF7, N-LASF46B und N-BAF4 gebildet wird, wobei Flintglas vorzugsweise die Glasreferenz N-SF14 ist, wobei diese Glasreferenzen der SCHOTT-Norm entsprechen.

7. Verfahren zur Herstellung einer polychromatischen optischen Messvorrichtung (1), die imstande ist, unter ionisierender Strahlung zu arbeiten, nach einem der Ansprüche 1 bis 6, wobei beim Schritt des Auswählens des Flintglases mindestens ein zweites optisches Flintglas, zweites Flintglas genannt, ausgewählt wird, wobei das Flintglas ein anderes Flintglas als das Flintglas unter den Gläsern der Gruppe von optischen Flintgläsern ist, bei denen der Mittelwert der durch Bestrahlung induzierten Abschwächung, RIA, im ersten Wellenlängenbereich kleiner als 0,25 dB/mm ist,

> wobei beim Schritt zum Bilden mindestens einer ersten optischen Komponente (21) aus Kronglas und mindestens einer zweiten optischen Komponente (22) aus Flintglas auch mindestens eine dritte optische Komponente (23) aus zweitem Flintglas gebildet wird, und
> wobei beim Schritt zum Vereinigen der ersten optischen Komponente (21) und der zweiten optischen Komponente (22), um ein optisches System (20) der optischen Messvorrichtung (1) zu bilden, die erste optische Komponente (21) und zweite optische Komponente (22) auch mit der dritten optischen Komponente (23) vereinigt werden, um das optische Messsystem (20) zu bilden.

8. Verfahren zur Herstellung einer polychromatischen optischen Messvorrichtung (1), die imstande ist, unter ionisierender Strahlung zu arbeiten, nach einem der Ansprüche 1 bis 7, wobei beim Schritt zum Auswählen des Kronglases das ausgewählte Kronglas ein reines Siliziumdioxidglas ist.

9. Polychromatische optische Messvorrichtung (1), die imstande ist, unter ionisierender Strahlung zu arbeiten, wobei die Vorrichtung umfasst:

> - ein Erkennungssystem (10), das imstande ist, unter ionisierender Strahlung zu arbeiten,
> - ein optisches System (20), das mit dem Erkennungssystem (10) vereinigt ist, wobei das optische System (20) umfasst:

>> * mindestens eine erste optische Komponente (21) aus optischem Kronglas, wobei das optische Kronglas Kronglas genannt wird, und
>> * mindestens eine zweite optische Komponente (22) aus optischem Flintglas, das Flintglas genannt wird,

>> wobei in dem optischen System (20) die erste und zweite optische Komponente (21, 22) vereinigt sind, um das optische System (20) zu bilden,
>> wobei die optische Messvorrichtung (20) **dadurch gekennzeichnet ist, dass** das Flintglas in einer Gruppe aus optischen Flintgläsern umfasst ist, die jeweils eine Cer-freie Zusammensetzung aufweisen, das Kronglas in einer Gruppe aus optischen Krongläsern umfasst ist, die jeweils eine Cer-freie Zusammensetzung aufweisen,
>> und dadurch, dass das Flintglas einen Mittelwert der durch Bestrahlung induzierten Abschwächung im ersten Wellenlängenbereich kleiner als 0,25 dB/mm aufweist, wobei der Mittelwert der durch Bestrahlung induzierten Abschwächung aus mindestens einer ersten repräsentativen Größe einer optischen Übertragung des Flintglases und mindestens einer zweiten repräsentativen Größe einer optischen Übertragung des Flintglases bestimmt wird,

wobei die erste repräsentative Größe einer optischen Übertragung einer Vorbestrahlungsübertragungsgröße in mindestens einem ersten Wellenlängenbereich von Interesse für eine Probe des Flintglases entspricht,

die zweite repräsentative Größe einer optischen Übertragung einer Nachbestrahlungsübertragungsgröße für die Probe des Flintglases entspricht, die nach Bestrahlung der Probe des Flintglases durch eine ionisierende Strahlung mit einer Dosis größer oder gleich 100 kGy erhalten wird.

10. Optische Messvorrichtung (1), die imstande ist, unter ionisierender Strahlung zu arbeiten, nach Anspruch 9, wobei der erste vorbestimmte Wellenlängenbereich zwischen 350 nm und 600 nm, vorteilhafterweise zwischen 400 nm und 550 nm und vorzugsweise zwischen 425 nm und 500 nm umfasst ist.

11. Optische Messvorrichtung (1) nach Anspruch 9 oder 10, wobei das Flintglas aus der Gruppe von Flintgläsern ausgewählt ist, die durch die Glasreferenzen N-SF2, N-SF6, N-SF10, N-SF14, N-SF66, N-LAF7, N-LASF46B und N-BAF4 gebildet wird, wobei das Flintglas vorzugsweise die Glasreferenz N-SF14 ist, wobei diese Glasreferenzen der SCHOTT-Norm entsprechen.

12. Optische Messvorrichtung (1) nach einem der Ansprüche 9 bis 11, wobei das optische System (20) weiter mindestens eine dritte Komponente umfasst, die aus einem anderen zweiten optischen Flintglas, zweites optisches Flintglas genannt, als dem Flintglas hergestellt ist, wobei das zweite Flintglas in der Gruppe von optischen Flintgläsern umfasst ist, die jeweils eine Cer-freie Zusammensetzung aufweisen,

und das zweite Flintglas den Mittelwert der durch Bestrahlung induzierten Abschwächung, RIA, im ersten Längenbereich kleiner als 0,25 dB/mm aufweist, wobei der Mittelwert der durch Bestrahlung induzierten Abschwächung, RIA.

13. Optische Messvorrichtung (1) nach einem der Ansprüche 9 bis 12, wobei das Kronglas reines Siliziumdioxidglas ist.

## Claims

1. A method for manufacturing a polychromatic optical measuring device (1) capable of operating under ionising radiation, the method comprising the following steps:

- selecting at least one first crown optical glass from a group of crown optical glasses each having a cerium-free composition, said selected crown optical glass being called crown glass,
- selecting at least one first flint optical glass from a group of flint optical glasses each having a cerium-free composition, said selected flint optical glass being called flint glass,
- providing a detection system (10) capable of operating under ionising radiation,
- forming at least one first optical component (21) made of crown glass and at least one second optical component (22) made of flint glass,
- combining the first optical component (21) and the second optical component (22) to form an optical system (20) of the optical measuring device (1),
- combining the detection system (10) and the optical system (20) to form the optical measuring device (1),

wherein the step of selecting the flint glass comprises the following substeps:

- measuring at least one first magnitude representative of an optical transmission in at least one first range of wavelengths of interest for a sample (110) of each glass from the group of flint optical glasses, said first magnitude representative of an optical transmission being called pre-irradiation transmission magnitude,
- irradiating each sample (110) with ionising radiation with a dose greater than or equal to 100 kGy,
- measuring at least one second magnitude representative of an optical transmission in the first range of wavelengths of interest for each sample (110), said second magnitude representative of an optical transmission being called the post-irradiation transmission magnitude,
- selecting the flint glass from the glasses of the group of flint optical glasses for which a mean value of the radiation-induced attenuation, RIA, in the first length range is less than 0.25 dB/mm, said mean value of the radiation-induced attenuation being determined from the first magnitude representative of a corresponding optical transmission and the second magnitude representative of a corresponding optical transmission.

2. The method for manufacturing a polychromatic optical measuring device (1) capable of operating under ionising radiation according to claim 1, wherein the mean value of the radiation-induced attenuation, RIA, is calculated from the following equation:

$$\overline{\mathrm{RIA}} = -\frac{10}{\mathrm{L}(\lambda 2 - \lambda 1)} \int\limits_{\lambda 1}^{\lambda 2} \log_{10}\left(\frac{\mathrm{I_{post}}(\lambda)}{\mathrm{I_{pre}}(\lambda)}\right) d\lambda$$

with L a thickness of the sample in millimetres, $\mathrm{I_{post}}(\lambda)$ and $\mathrm{I_{pre}}(\lambda)$ corresponding to a transmitted signal intensity corresponding respectively to the post-irradiation transmission magnitude and to the pre-irradiation transmission magnitude at the wavelength $\lambda$, and $\lambda 1$ and $\lambda 2$ being respectively the wavelength of the minimum and maximum limit of the first wavelength range.

3. The method for manufacturing a polychromatic optical measuring device (1) capable of operating under ionising radiation according to claim 1 or 2, wherein the first predetermined wavelength range is comprised between 350 nm and 600 nm, advantageously between 400 nm and 550 nm and, preferably, between 425 nm and 500 nm.

4. The method for manufacturing a polychromatic optical measuring device (1) capable of operating under ionising radiation according to claims 1 to 3, wherein during the substep of irradiating each sample with ionising radiation, the ionising radiation is X-ray or gamma radiation.

5. The method for manufacturing an optical measuring device (1) capable of operating under ionising radiation according to any one of claims 1 to 4, wherein the pre-irradiation transmission magnitude and the post-irradiation transmission magnitude are selected from the group comprising an intensity of a transmitted optical signal, a transmission rate or coefficient and an absorption rate or coefficient.

6. The method for manufacturing a polychromatic optical measuring device (1) capable of operating under ionising radiation according to any one of claims 1 to 5, wherein the flint glass is selected from the group of flint glasses consisting of the glass references N-SF2, N-SF6, N-SF10, N-SF14, N-SF66, N-LAF7, N-LASF46B and N-BAF4, the flint glass preferably being the glass reference N-SF14, these glass references being compliant with the SCHOTT standard.

7. The method for manufacturing a polychromatic optical measuring device (1) capable of operating under ionising radiation according to any one of claims 1 to 6, wherein during the step of selecting the flint glass, at least one second flint optical glass, called second flint glass, is selected, said flint glass being a flint glass other than the flint glass from the glasses of the group of flint optical glasses for which the mean value of the radiation-induced attenuation, RIA, in the first wavelength range is less than 0.25 dB/mm,

   wherein during the step of forming at least one first optical component (21) made of crown glass and at least one second optical component (22) made of flint glass, at least one third optical component (23) made of second flint glass is also formed, and
   wherein during the step of combining the first optical component (21) and the second optical component (22) to form an optical system (20) of the optical measuring device (1), the first optical component (21) and second optical component (22) are also combined with the third optical component (23) to form the optical measuring system (20).

8. The method for manufacturing a polychromatic optical measuring device (1) capable of operating under ionising radiation according to any one of claims 1 to 7, wherein during the step of selecting the crown glass, the selected crown glass is a pure silica glass.

9. A polychromatic optical measuring device (1) capable of operating under ionising radiation, said device comprising:

   - a detection system (10) capable of operating under ionising radiation,
   - an optical system (20) associated with the detection system (10), the optical system (20) comprising:

      • at least one first optical component (21) made of crown optical glass, said crown optical glass being called crown glass, and

• at least one second optical component (22) made of a flint optical glass, called flint glass,

in which optical system (20), the first and second optical components (21, 22) are combined to form said optical system (20),

the optical measuring device (20) **being characterised in that** the flint glass is comprised in a group of flint optical glasses each having a cerium-free composition,

the crown glass being comprised in a group of crown optical glasses each having a cerium-free composition,

and **in that** the flint glass has a mean value of the radiation-induced attenuation in the first wavelength range which is less than 0.25 dB/mm, said mean value of the radiation-induced attenuation being determined from at least one first magnitude representative of an optical transmission of said flint glass and at least one second magnitude representative of an optical transmission of said flint glass,

the first magnitude representative of an optical transmission corresponding to a pre-irradiation transmission magnitude in at least one first range of wavelengths of interest for a sample of the flint glass,

the second magnitude representative of an optical transmission corresponding to a post-irradiation transmission magnitude for the sample of the flint glass obtained after irradiation of the sample of flint glass by ionising radiation with a dose greater than or equal to 100 kGy.

10. An optical measuring device (1) capable of operating under ionising radiation according to claim 9, wherein the first predetermined wavelength range is comprised between 350 nm and 600 nm, advantageously between 400 nm and 550 nm and, preferentially, between 425 nm and 500 nm.

11. The optical measuring device (1) according to claim 9 or 10, wherein the flint glass is selected from the group of flint glasses consisting of the glass references N-SF2, N-SF6, N-SF10, N-SF14, N-SF66, N-LAF7, N-LASF46B and N-BAF4, the flint glass preferably being the glass reference N-SF14, these glass references being compliant with the SCHOTT standard.

12. The optical measuring device (1) according to any one of claims 9 to 11, wherein the optical system (20) further comprises at least one third component made of a second flint optical glass called second flint optical flint glass other than the flint glass, the second flint glass being comprised in the group of flint optical glasses each having a cerium-free composition,

and the second flint glass has the mean value of the radiation-induced attenuation, RIA, in the first length range less than 0.25 dB/mm, called mean value of the radiation-induced attenuation, RIA.

13. The optical measuring device (1) according to any one of claims 9 to 12, wherein the crown glass is a pure silica glass.

FIG. 1

FIG. 2

FIG. 3

EP 3 999 895 B1

FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 105676451

**Littérature non-brevet citée dans la description**

- **VINCENT GOIFFON**. Toward Multi-MGy / Grad Radiation Hardened CMOS Image Sensors for Nuclear Applications. *conférence internationale « International Image Sensor Workshop (IISW 2015)*, 08 June 2015

- **VINCENT GOIFFRON**. *IEEE Transactions on nuclear science*, December 2015, vol. 62 (6), 2956-2964